# EUROPEAN PATENT SPECIFICATION

(11) **EP 0 996 396 B1**
(45) Date of publication and mention of the grant of the patent: **15.09.2004**
(21) Application number: 98932507.1
(22) Date of filing: 09.07.1998
(51) Int. Cl.: A61F 5/01

(54) **WRIST-BAND FOR THE PREVENTION AND THE TREATMENT OF THE CARPAL TUNNEL SYNDROME AND ITS POSITIONING OPERATING MODE**
HANDGELENKBAND ZUR VERMEIDUNG UND BEHANDLUNG DES KARPALTUNNELSYNDROMS
BANDEAU DE POIGNET PERMETTANT DE TRAITER ET DE PREVENIR LE SYNDROME DU CANAL CARPIEN ET PROCEDE D'UTILISATION ET DE POSITIONNEMENT DE CE BANDEAU

(30) Priority: 09.07.1997 IT FI970164
(43) Date of publication of application: 03.05.2000
(73) Proprietor: Massi, Gabriele, 52027 San Giovanni Valdarno (IT); Massi, Stefano, 52027 San Giovanni Valdarno (IT)
(72) Inventor: Massi, Gabriele, 52027 San Giovanni Valdarno (IT); Massi, Stefano, 52027 San Giovanni Valdarno (IT)
(74) Representative: Gustorf, Gerhard, Dipl.-Ing.
(86) International application number: PCT/IT1998/000193
(87) International publication number: WO 1999/002111

(56) References cited:
- WO-A1-97/23176
- US-A- 5 014 689
- US-A- 5 376 066
- US-A- 5 417 645
- US-A- 5 538 501

## Description

The present invention belongs to the field of the othopedic remedies and it relates to a wrist-band adjuvant for the prevention and the therapy of the carpal tunnel syndrome.

Problems of the neuropaties of the wrist are currently well known whose symptoms are often due to factors such as cervical arthrosis or to circulatory insufficiency and submitted to specialistic treatment late or often treated in an inappropriate manner.

To better focus the problem, the movements of the human wrist according to its morphology are here described. For this purpose it is necessary to consider that in the carpiradialis and carpimedian articulations movements which cause the changing of position of the hand according to the forearm are accomplished; such movements can be bending movements, extension movements, radial and cubital inclination movements, circling and rotation movements; accomplishing the bending movement, the palmar face of the hand bends itself according to the front surface of the forearm, whereas for the extension movement, the dorsal face of the hand bends itself according to the rear surface of the forearm; accomplishing the cubital inclination movement or adduction movement, the hand bends itself towards the cubital side of the forearm and in the radial inclination movement or abduction movement the hand bends itself towards the radial side. The sequence and the combination of the above described movements cause the circling, whereas the rotation is obtained by the movement of the hand on its own axis.

All these movements are accomplished through the the carpiradialis and carpimedian articulations and through the flexor and extensor muscles of the forearm and of the hand, which are innervated also by the median nerve which crosses the carpal canal constituted by the rigid osseous sulcus of the carpal bones on which the traverse ligament is stretched.

The neuropathy which regards the present invention arises from the unbalanced relation between the cavity constituting the carpal canal and its sheath (containment) and the respective nerve (content) as well as from endogenous and exogenous factors which may cause such degenerative process; exogenous factors which may be the most different, among which the following ones may be indicated: inflammatory processes (rheumatoid arthritis), fractures of the wrist and of osseous corn, acromegaly (abnormal swelling of the boncs), pregnancy etc.
From the repeated observation of patients affected by such pathology, it has been noted a very frequent etiology due to a particular exogenous factor, that is the frequent constrained position of extension and bending of the wrist which for the forced mechanical motion arouses the suffering of the median nerve caused by the increase of the pression that occurs in the carpal canal. Consequences of the pathology are irritative disorders, diffuse pain, paresthesia (tingling), as well as a further loss of the contractile strength of some muscles that, in certain cases, leads to the atrophy of the muscles themselves. The most usual treatment for this pathology is of conservative type, wherein anti-inflammatory substances are used even with infiltrations along the nerve; such treatment causes relevant incoveniences which are even increased considering that some histological reports indicate the damage caused by such medicament and that the eventual advantage obtained is temporary. A first alternative to anti-inflammatory medicines is constituted by the use of wrist splints obtained by using metal splints having a padding, such splints preventing movements of hyperextension and bending relieve the suffering of the nerve with the incoveniences, however, caused by the necessity to arrange the wrist-splint "ad hoc and in situ" by a technician, as well as caused by limitation that the splint applied to one wrist or to both wrists causes to the daily and working activities of the patient, as well as by the impossibility to remove it by the patient.

On the same principle of the splints, other orthopedic remedies are proposed which are basically constituted by armbands worn by the patient with the purpose of limiting as much as possible movements which cause the sliding of the nerve along the carpal canal. Such remedies do not allow the normal use of the hand and their correct positioning is not simple; furthermore they are not born by the patients for adequate periods of time necessary for the treatment of the pathology.

However it is important to consider that such remedies are sometimes used in an improper manner for the carpal tunnel syndrome because for this pathology it is fundamental to limit the bending-extention of the wrist without blocking completely its movements, because the block of the wrist causes the suffering of the nerve, as shown by the fact of the arising of the carpal tunnel syndrome after the plaster of the wrist due to a fracture and during the night rest.

An attempt to resolve the problem has been made by the same applicants who in WO 97/23176 A1 describe a removable wrist-band adjuvant in the carpal tunnel syndrome theraphy, consisting in an armband for containing or preventing the bending movements, extension movements, radial and cubital inclination movements and movements of rotation of the carpiradialis and carpiulnaris articulations of the wrist, allowing the opposing of the thumb and the normal use of the hand. Such armband, padded and countershaped according to the wrist and partially to the hand, is basically constituted by a shell having a rigid but padded frame presenting two sides the smaller of which, by using the wrist-band, is turned towards the forearm and the larger is turned towards the digits of the hand and means for allowing the blockage to the wrist.

Such embodiment, as well as having some constructive difficulties, presents the inconvenience that, once the patient has worn the wrist-band, it does not remain in the requested position for obtaining the therapeutic purpose, but it slides along the forearm; it presents furthermore the non-perfect containment of the wrist according to the bending movements.

The main aim of the present invention is to prevent and obviate to the carpal tunnel syndrome without using pharmacological medicaments and by using a device which can be positioned directely by the patient without the help of a technician.

An other aim is to allow the use of the device by the patient compatibly with its own daily activities and to obtain the easy positioning and removal of the remedy.

A further aim is to obtain a remedy which, when in use applied on the wrist, even if it contains the bending and extention movements, does not prevent to the patient to carry out its own working activities and does not block completely the hand causing the consequent suffering of the median nerve.

Not least aim of the invention is to obtain a device of easy and inexpensive manufacturing without using specialized work-people and which is reproducible on an industrial scale without the use of relevant equipment or sophisticated technologies and consequently manufactured in relevant quantities proportioned to the high number of current pathologies and to the potential pathologies arising in future.

These and other aims are achieved by a wrist-band as set forth in claim 1.

Further characteristics and advantages of the invention will more clearly arise from the description of a preferred but not exclusive embodiment of the wrist-band shown as an indicative and not limitative way in the attached drawings in which:
- figure 1 shows a dorsal view of the open wrist-band;
- figure 2 shows an internal view of the open wrist-band;
- figure 3 shows a cross section view of a central portion;
- figure 4 shows a general view of the wrist-band in position of use;
- figure 5 shows a general view of the wrist-band in a step of positioning on the wrist.

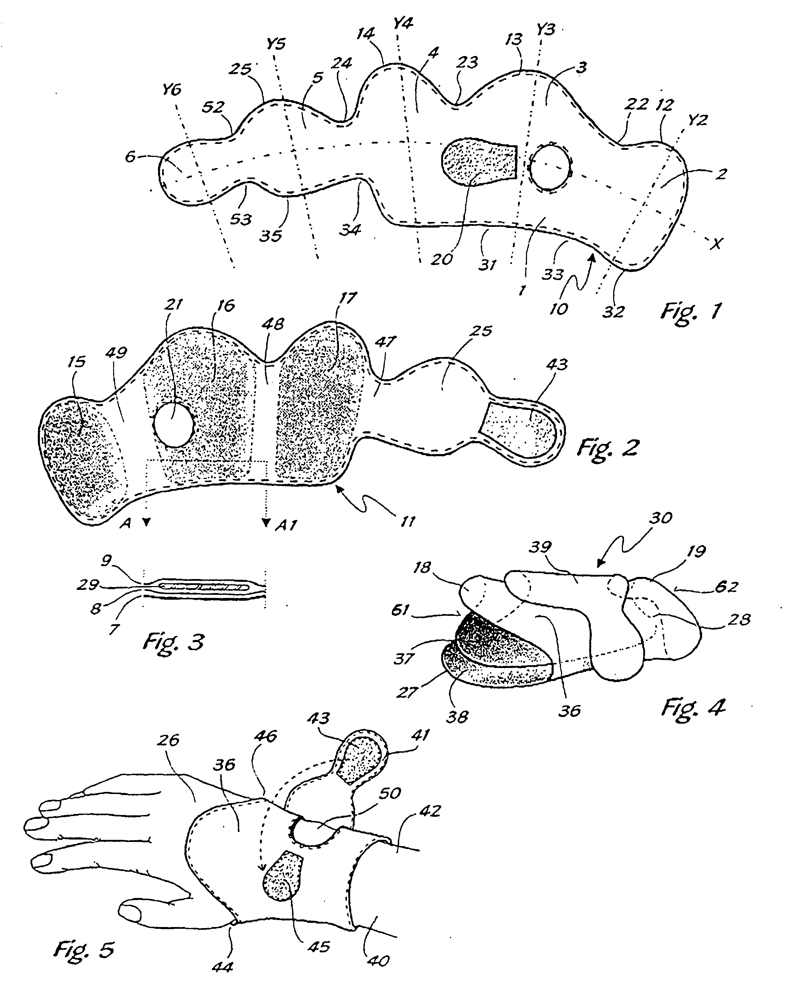

In figures 1 and 2, it is shown a soft and flexible multi-layer band in an opened position, generally indicated by the number 10; in figure 1 it is shown the external face 1 and in figure 2 the internal face 11 with the representation of the areas which present reinforcements; the band develops according to a curved longitudinal axis x and it is ideally divided in five portions 2, 3, 4, 5, 6 developping themselves according to respective transversal axes y2, y3, y4, y5, y6 placed in a fan-shape way. The first three portions (2, 3, 4) each adjacent to the other ones, constitute the main body presenting the superior sides, respective 12, 13 and 14 rounded, being the sides 12 and 13 connected through the groove 22 and the sides 13 and 14 connected through the groove 23; the inferior rounded side 32 of the first portion 2 is connected through the groove 33 to the rectilinear inferior side 31, the same for the portions 3 and 4. The side of the head of the portion 2 is also rounded and it completes the ovoidal shape of said portion; the portion 4 is connected to the portion 5 upwards through the groove 24 and downwards through the groove 34. The portion 5 which presents the superior side 25 rounded as well as the inferior side 35, is connected through the superior groove 52 and the inferior groove 53 to the terminal portion 6 which is tongue-shaped and has smaller size than the others.

The external face 1 has between portions 3 and 4, an area 20 having a shape like that of the tongue-shaped portion 6, fit for the positioning of fastening means when such band has been wrapped round the wrist like an armband.

The band 10, as shown in figure 3, is constituted by a first external layer 7, of a soft and flexible material such as leather or imitation leather or an other washable and waterproof material, by a second internal layer 8 of gutta-percha or other flexible insulating material with a function of structural frame, by a third external layer 9 of a textile, non-toxic material, transpiring and however washable, fit for being in touch with the epidermis of the wrist. Three areas 15, 16 and 17 are approximately coincident with the portions 2, 3 and 4, on which, between the second layer 8 and the third layer 9, the same number of leather stiffening portions 29 are placed, having such a thickness to allow them to be stiff for the containment but at the same time flexible for being adapted according to the anatomic morphology of the wrist: the use of the leather may be substitute by a plastic material or by a agglomerated natural fiber having the mechanical characteristics and resistance for obtaining the same purpose. In the center of the second portion 3 a circular eyelet 21 is provided, passing the four layers.

The final embodiment of the wrist-band device 30 as shown in figure 4 will be obtained by the folding of the band 10 according to the areas having a smaller resistance 47, 48 and 49 which allow to model said band giving it a mainly tubular outline and adapting said band according to the morphology of the wrist, obtaining however a device which will have a first major opening 61 turned towards the digits and a second smaller opening 62 turned towards the forearm, said device being consequently funnel-shaped.

In figure 5 the wrist-band object of the invention is shown when it has been positioned on the wrist of a right hand wherein, for the necessary correct positioning, the posterior or dorsal face 26 of the hand will have to be turned upwards, on said face the lap 36 of the wrist-band 10 with the edge 18 of the band 10 constituted by the side 13 of the portion 3 rests, turned towards the digits of the hand, whereas the edge 19 constituted by the side 31 of the same portion is turned towards the forearm and the eyelet 21 is placed corresponding to the protusion 50 constituted by the capital of the ulna; then the lap 37 will have to be positioned for being coincident with the front or palmar face of the hand, with the edge 27 turned towards the digits and with the opposite edge 28 turned towards the forearm and in this way tightly maintained during the successive step in which the lateral face 40 of the forearm will have to be leaned to the thorax of the patient with the purpose of maintaining the wrist-band 10 in its position during the successive steps. Proceeding, with a rotating movement towards the medial side 42 of the forearm, the lap 38 will have to be passed under the lap 37 exactely overlapping itself to it as to carry out - once the steps of positioning are finished - a consistent blockage of the bending movements of the wrist; consequently the lap 39 will lay upon the lap 36 and it will close the eyelet 21 in which the profusion 50 of the ulna has been already inserted; at this point, the next step will be to grasp the tongue-shaped edge 41 carrying out the regulation of the whole tubular set of the wrist-band, which will be obtained through the overlapping of the fastening means 43 present on the wrist-band, to the corresponding fastening means 45 present on the external face of the lap 36.

The correct positioning of the wrist-band is particularly relevant for achieving the therapeutic purpose. The main rule consists in the exact axial alignment of the lap 36 with the laps 37 and 38; consequently on the lateral face of the wrist, in such position, the groove 44 constituted by the folding of the band according to the area of smaller resistance 49, will have to be in such a position to house the most developed raised area of the hand, corresponding to the basis of the thumb and determined by the fleshy mass of the three muscles, brief flexor of the thumb, brief abductor of the thumb and opponent of the thumb, considering furthermore that such raised area (known as thenar eminence) has an ovoidal elongate shape with the larger pole turned towards the thumb and consequently countershaped to the afore said groove; on the opposite side, the groove 46 constituted by the folding of the band according to the area of smaller resistance 48. will have to be in such a position to house the minor projecting raised area (known as ipothenar eminence) of elliptical elongate shape which corresponds to the little finger of the hand and it is constituted by the abductor, brief flexor and opponent muscles of the little finger. An other relevant rule is based on the correct and stable positioning on the wrist which will be obtained when the eyelet 21 present on the band 10 will be coincident with the capital of the ulna (malleolus) 50 and, as well as to determine a fixed positioning will avoid the possibile excursions of the wrist-band both on transversal and longitudinal direction.

The advantages of such a wrist-band/device are evident, due to the immediate and easy possibility to position it directly on the wrist with simple movements carried out directly by the patient, as well as it is simple to obtain a range of adjustments and intermediate tightening regulations which allow a simple adapting of the wrist-band to the different daily activities (more tightened when the wrist is at rest, more loosen in the other cases).

Its simple and pleasant shape makes it well-accepted among the plurality of the patients without difference of sex and activities, because it allows a continuous even prolunged in time use that is necessary for obtaining the therapeutic advantage.

## Claims

1. Wrist-band for the prevention and the treatment of the carpal tunnel syndrome for allowing limited movements of the wrist of the user according to bending-extension movements, **characterized by** the fact that it is constituted by a multi-layer soft and flexible band (10) fit for being placed on a short portion between the hand and the forearm, said portion corresponding to the carpiulnaris articulation of the user, said band (10) comprising five adjacent portions aligned:
- a first terminal portion (2) approximately coincident with the volar face of the wrist presenting three rounded sides of which one is the terminal side and the superior (12) and the inferior (32) connected through two respective grooves (22, 33) to
- a second central portion (3) adjacent to the first terminal portion (2) approximately coincident with the dorsal face of the wrist, said portion (3) presenting two opposite sides of which the superior rounded side (13) is connected through a groove (23) to the adjacent third portion (4) and the inferior rectilinear side (31) is further connected to
- the third central portion (4) adjacent to the second portion (3), fit for overlapping the first one (2), said third portion (4) presenting two opposite sides of which the superior (14) is rounded and the inferior is a continuation of the inferior rectilinear side (31) of the second portion (3), said third portion (4) is further connected to
- a fourth portion (5) adjacent to the third portion (4) and of smaller size having the two opposite superior (25) and inferior (35) sides both rounded and connected through two further grooves (52, 53) to
- a fifth terminal tongue-shaped portion (6) constituted by three consecutive rounded sides, presenting on the internal face fastening means (43) constituted by a grasping area obtained through a fastening/removal material, wherein the opposite and terminal sides of the band are convexly rounded and the five adjacent portions (2, 3, 4, 5, 6) are aligned along a curved longitudinal axis (x), the single portions developping themselves according to respective transversal axes (y2, y3, y4, y5, y6) placed in a fan-shaped way.

2. Wrist-band according to claim 1, **characterized in that** between the second (3) and the third portion (4) it presents on the external face fastening means (45) constituted by a grasping area obtained through a fastening/removal material.

3. Wrist-band according to claim 1 or 2, **characterized in that** in the center of the second portion (3) a circular passing eyelet (21) is provided.

4. Wrist-band according any of the preceding claims, **characterized in that** the band presents three layers (7, 8, 9).

5. Wrist-band according to claim 4, **characterized in that** the first external layer (7) is made of leather.

6. Wrist-band according to claim 4, **characterized in that** the first external layer (7) is made of imitation leather or other washable and waterproof material.

7. Wrist-band according to claim 4, **characterized in that** the second internal layer (8) is made of guttha-percha.

8. Wrist-band according to claim 4, **characterized in that** the second internal layer (8) is made of a flexible and insulating material having structural supporting function.

9. Wrist-band according to claim 4, **characterized by** the fact that the third external layer (9) touching the epidermis of the wrist is carried out in a textile, non-toxic, transpiring and washable material.

10. Wrist-band according to any of the preceding claims, **characterized in that** between the second (8) and the third layer (9) it presents three leather stiffening portions (29) corresponding to the leaning areas of the first (2), second (3) and third (4) portions.

11. Wrist-band according to claim 10, **characterized in that** said stiffening portions (29) are made in any plastic material or agglomerated natural fiber material which can substitute the leather as per mechanical resistance and structural characteristics.

12. Wrist-band according to any of the preceding claims, **characterized in that** it is carried out by way of folding the band (10) according to its areas (49, 48) having smaller resistance placed between the first (2) and the second portion (3) and between the second (3) and the third portion (4) respectively, said folding allowing a final tubular and funnel-shaped outline.

## Patentansprüche

1. Handgelenkband zur Vermeidung und Behandlung des Karpaltunnelsyndroms derart, daß der Benutzer begrenzte Bewegungen des Handgelenks mit Biege-Streck-Anteilen durchführen kann, **dadurch gekennzeichnet, daß** dieses aus einem weichen und flexiblen, mehrschichtigen Band (10) besteht, das so ausgebildet ist, daß es an einem kurzen, zwischen der Hand und dem Unterarm liegenden Abschnitt angelegt werden kann, der das Handwurzelgelenk des Benutzers einschließt, wobei das Band (10) fünf aufeinander folgende und aneinander angrenzende Abschnitte hat:
- einen ersten Endabschnitt (2), der im wesentlichen auf der volaren Fläche des Handgelenks liegt und drei abgerundete Seiten hat, von denen eine die Abschlußkante bildet, während die obere Seite (12) und die untere Seite (32) über zwei entsprechende Einbuchtungen (22, 33) verbunden sind mit
- einem zweiten, inneren Abschnitt (3), der an den ersten Abschnitt (2) anschließt, im wesentlichen auf der dorsalen Fläche des Handgelenks liegt und zwei einander gegenüberliegende Seiten hat, von denen die obere, abgerundete Seite (13) über eine Einbuchtung (23) an den folgenden, dritten Abschnitt (4) anschließt, während die untere, gerade Seite (31) verbunden ist mit
- dem dritten, inneren Abschnitt (4), der auf den zweiten Abschnitt (3) folgt und so ausgebildet ist, daß er den ersten Abschnitt (2) überlappt, wobei dieser dritte Abschnitt (4) zwei einander gegenüberliegende Seiten hat, von denen die obere Seite (14) abgerundet ist, während die untere Seite eine Fortsetzung der unteren, geraden Seite (31) des zweiten Abschnitts (3) darstellt, wobei der dritte Abschnitt (4) verbunden ist mit
- einem vierten Abschnitt (5), der auf den dritten Abschnitt (4) folgt, schmäler als dieser ist und zwei einander gegenüberliegende, abgerundete Seiten hat, nämlich eine obere Seite (25) und eine untere Seite (35), die beide über zwei weitere Einbuchtungen (52, 53) verbunden sind mit
- einem fünften, am Ende liegenden, zungenförmigen Abschnitt (6), der von drei ineinander übergehenden, abgerundeten Seiten begrenzt ist und auf seiner Innenfläche Fixiermittel (43) aufweist, die durch eine Klettfläche aus Festhalte-/Freigabematerial gebildet sind, wobei die einander gegenüberliegenden Seiten sowie die das Band abschließende Seite konvex abgerundet sind und die fünf aneinander anschließenden Abschnitte (2, 3, 4, 5, 6) zu einer bogenförmigen Längsachse (x) des Bandes ausgerichtet sind und sich die einzelnen Abschnitte über entsprechende Querachsen (y2, y3, y4, y5, y6) erstrecken, die fächerförmig angeordnet sind.

2. Handgelenkband nach Anspruch 1, **dadurch gekennzeichnet, daß** zwischen dem zweiten Abschnitt (3) und dem dritten Abschnitt (4) auf der Außenfläche Fixiermittel (45) angebracht sind, die aus einer Klettfläche mit Festhalte-/Freigabematerial bestehen.

3. Handgelenkband nach Anspruch 1 oder 2, **dadurch gekennzeichnet, daß** in die Mitte des zweiten Abschnittes (3) ein rundes, durchgehendes Fenster (21) eingearbeitet ist.

4. Handgelenkband nach einem der vorhergehenden Ansprüche, **dadurch gekennzeichnet, daß** das Band drei Schichten (7, 8, 9) enthält.

5. Handgelenkband nach Anspruch 4, **dadurch gekennzeichnet, daß** die erste, außenliegende Schicht (7) aus Leder besteht.

6. Handgelenkband nach Anspruch 4, **dadurch gekennzeichnet, daß** die erste, außenliegende Schicht (7) aus Lederimitation oder einem anderen Material besteht, das waschbar und wasserdicht ist.

7. Handgelenkband nach Anspruch 4, **dadurch gekennzeichnet, daß** die zweite, innere Schicht (8) aus Guttapercha besteht.

8. Handgelenkband nach Anspruch 4, **dadurch gekennzeichnet, daß** die zweite, innere Schicht (8) aus einem flexiblen, isolierenden Material besteht, das eine konstruktive Stützfunktion hat.

9. Handgelenkband nach Anspruch 4, **dadurch gekennzeichnet, daß** die dritte, äußere Schicht (9), welche in Berührung mit der Haut des Handgelenks kommt, aus einem waschbaren, nicht toxischen, atmungsfähigen Werkstoff besteht.

10. Handgelenkband nach einem der vorhergehenden Ansprüche, **dadurch gekennzeichnet, daß** zwischen die zweite Schicht (8) und die dritte Schicht (9) drei Versteifungsabschnitte (29) aus Leder eingesetzt sind, welche im Bereich der Stützflächen des ersten (2), zweiten (3) bzw. dritten (4) Abschnitts liegen.

11. Handgelenkband nach Anspruch 10, **dadurch gekennzeichnet, daß** die Versteifungsabschnitte (29) aus Kunststoff oder einem gepressten Naturfaserwerkstoff bestehen, der hinsichtlich der mechanischen Festigkeit und der Struktureigenschaften als Lederersatz dienen kann.

12. Handgelenkband nach einem der vorhergehenden Ansprüche, **dadurch gekennzeichnet, daß** dieses im Bereich der Flächen (49, 48) mit geringerer Festigkeit, die zwischen dem ersten Abschnitt (2) und dem zweiten Abschnitt (3) bzw. dem zweiten Abschnitt (3) und dem dritten Abschnitt (4) liegen, abgeknickt ist, wodurch die endgültige, rohrartige Trichterform gebildet wird.

## Revendications

1. Bandage de poignet pour éviter et soigner le syndrome du tunnel carpien, de telle sorte que l'utilisateur ne puisse exécuter que des mouvements limités de flexion et d'extension du poignet, **caractérisé par le fait qu'**il est constitué d'une bande (10) multi-couches en matériau doux et flexible dont la forme lui permet de trouver place dans la courte portion située entre la main et l'avant-bras correspondant à l'articulation carpienne de l'utilisateur, ladite bande (10) comprenant cinq parties adjacentes successives :
- une première partie d'extrémité (2) s'appliquant essentiellement sur la face palmaire de l'articulation carpienne et comprenant trois cotés arrondis dont l'un forme le bord de l'extrémité, tandis que le côté supérieur (12) et le côté inférieur (32) sont réunis par deux rétrécissements (22, 33)
- à une deuxième partie centrale (3) adjacente à la première partie (2), recouvrant essentiellement la face dorsale de l'articulation carpienne et qui possède deux côtés opposés, le côté supérieur arrondi (13) étant rattaché par un rétrécissement (23) à une troisième partie (4) adjacente, tandis que le côté inférieur (31) non courbe est rattaché
- à une troisième partie centrale (4) qui fait suite à la deuxième partie (3) et qui est configurée de telle sorte qu'elle vient recouvrir la première partie (2), cette troisième partie (4) possédant deux côtés opposés, le côté supérieur (14) étant arrondi, tandis que le côté inférieur représente la suite du côté (31) non courbe de la deuxième partie (3), cette troisième partie (4) étant rattachée
- à une quatrième partie (5) qui fait suite à la troisième partie (4), est plus étroite que celle-ci et possède deux côtés arrondis opposés, un côté supérieur (25) et un côté inférieur (35), les deux étant rattachés par deux autres rétrécissements (52, 53)
- à une cinquième partie (6) en forme de languette qui constitue l'extrémité et qui est limitée par trois côtés arrondis consécutifs et présente sur sa face intérieure des moyens de fixation (43) formés d'une surface auto-accrochante en matériau de maintien/de relâchement, les côtés opposés ainsi que le côté d'extrémité de la bande présentant un arrondi convexe, tandis que les cinq parties successives (2, 3, 4, 5, 6) du bandage sont alignées suivant un axe longitudinal (x) incurvé, et que les parties individuelles se développent suivant des axes transversaux (y2, y3, y4, y5,y6) placés en éventail.

2. Bandage de poignet selon la revendication 1, **caractérisé par le fait qu'**entre la deuxième partie (3) et la troisième partie (4) sont prévus des moyens de fixation (45) constitués par une surface auto-accrochante formée d'un matériau de maintien / de relâchement.

3. Bandage de poignet selon la revendication 1 ou 2, **caractérisé par le fait qu'**une fenêtre (21) est pratiquée de part en part au milieu de la deuxième partie (3).

4. Bandage de poignet selon une des revendications précédentes, **caractérisé par le fait que** le bandage présente trois couches (7, 8, 9).

5. Bandage de poignet selon la revendication 4, **caractérisé par le fait que** la première couche extérieure (7) est en cuir.

6. Bandage de poignet selon la revendication 4, **caractérisé par le fait que** la première couche extérieure (7) est en similicuir ou en un autre matériau lavable et étanche à l'eau.

7. Bandage de poignet selon la revendication 4, **caractérisé par le fait que** la deuxième couche intérieure (8) est en gutta-percha.

8. Bandage de poignet selon la revendication 4, **caractérisé par le fait que** deuxième couche intérieure (8) est en matériau souple, isolant et possédant une fonction de support de structure.

9. Bandage de poignet selon la revendication 4, **caractérisé par le fait que** la troisième couche extérieure (9), qui est en contact avec la peau du poignet, est en matériau lavable, non toxique et perméable à l'air.

10. Bandage de poignet selon une des revendications précédentes, **caractérisé par le fait qu'**entre la deuxième couche (8) et la troisième couche (9) sont intercalées trois parties de renforcement (29) en cuir placées dans la zone de la surface de support de la première partie(2), de la deuxième partie (3) et de la troisième partie (4).

11. Bandage de poignet selon la revendication 10, **caractérisé par le fait que** les parties de renforcement (29) sont en matière plastique, ou dans un matériau en fibres naturelles pressées, susceptiple de pouvoir remplacer le cuir quant aux propriétés de résistance mécanique et aux caractéristiques structurelles.

12. Bandage de poignet selon une des revendications précédentes, **caractérisé par le fait que**, dans la zone de plus faible stabilité des surfaces (49, 48) placées entre la première partie (2) et la deuxième partie (3), ou entre la deuxième partie (3) et la troisième partie (4), celui-ci est incurvé, ce qui lui donne finalement une forme tubulaire d'entonnoir.
